# EUROPÄISCHE PATENTSCHRIFT

(11) **EP 1 470 813 B1**
(45) Veröffentlichungstag und Bekanntmachung des Hinweises auf die Patenterteilung: **27.09.2006**
(21) Anmeldenummer: 04101659.3
(22) Anmeldetag: 21.04.2004
(51) Int. Cl.: A61Q 5/02, A61K 8/46, A61K 8/31, A61K 8/06, A61Q 19/10, A61K 8/81

(54) **Reinigungsemulsion mit hohem Fettgehalt**
High fat cleansing emulsion
Emulsion de nettoyage à taux de matière grasse élevés

(30) Priorität: 24.04.2003 DE 10318526
(43) Veröffentlichungstag der Anmeldung: 27.10.2004
(73) Patentinhaber: Beiersdorf AG, 20245 Hamburg (DE)
(72) Erfinder: Kohut, Michaela, 20257, Hamburg (DE); Kauffeldt, Martin, 22307 Hamburg (DE); Rohde, Olaf, 22880, Wedel (DE); Ruppert, Stephan, 20259 Hamburg (DE)

(56) Entgegenhaltungen:
- WO-A-96/37595
- WO-A-98/55093
- DD-A- 236 014
- DE-A- 19 714 829
- US-A- 612 954
- US-A- 5 346 886
- US-A- 5 869 070
- US-A- 6 159 483

## Beschreibung

Die vorliegende Erfindung betrifft eine kosmetische und/oder dermatologische Reinigungsemulsion mit hohem Fettgehalt und geringen Mengen an Verdickungsmitteln.

Der Wunsch nach einem sauberen und gepflegten Äußeren ist wohl so alt wie die Menschheit. Unreine Haut und ein ungepflegtes Haarkostüm bieten idealen Nährboden und Heimstatt für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Kosmetische und/oder dermatologische Reinigungspräparate werden in aller Regel in Form eines Schaums mit Wasser auf die zu reinigenden Körperpartien aufgetragen. Basis fast aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Die hohe Reinigungsleistung tensidhaltiger Reinigungszubereitungen insbesondere gegenüber Lipiden, birgt jedoch auch eine Reihe von dermatologischen Nachteilen in sich: Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bades und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind (sogenannte Feuchthaltemittel oder Moisturizer). Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut.

Es ist verständlich, dass waschaktive Tenside, die Haut und Haar von fettigen und wasserlöslichen Schmutzbestandteilen reinigen sollen, auch eine entfettende Wirkung auf die normalen Hautlipide haben. Bei jeder Hautreinigung werden in unterschiedlichem Maß auch interkorneozytäre Lipide und Sebumbestandteile entfernt. Das bedeutet, dass der natürliche Wasser-Lipid-Mantel der Haut bei jedem Waschvorgang mehr oder weniger gestört wird. Dies kann besonders bei extremer Entfettung zu einer kurzzeitigen Veränderung der Barrierefunktion der Haut führen, wobei selbstverständlich auch der jeweilige Zustand der behandelten Hautregion auf die dargestellten Veränderungen von erheblichem Einfluss ist. Beispielsweise kann die Hautdicke, die Anzahl der Talg- und Schweißdrüsen sowie die damit verbundene Empfindlichkeit erheblich variieren.

Grundsätzlich gilt dementsprechend als Forderung an waschaktive Tenside, dass sie biologisch möglichst inaktiv sind, um unerwünschte Nebenwirkungen zu vermeiden. Sie sollen ihre reinigende Wirkung bei optimaler Milde, bester Hautverträglichkeit und geringer Entfettung entfalten.

Es hat daneben aber auch nicht an Versuchen gefehlt, geeignete Reinigungszubereitungen zu finden, welche die Haut bei guter Reinigungsleistung gleichzeitig regenerieren bzw. "rückfetten". Allerdings bleibt die erzielte Leistung häufig hinter der erwarteten zurück, so dass der Anwender in aller Regel auf separate Pflegeprodukte zurückgreifen muss, welche nach der Reinigung auf die Haut aufgetragen werden und auf dieser verbleiben (sogenannte "leave-on" Produkte).

Es sind nach dem Stande der Technik eine Reihe von Reinigungszubereitungen, beispielsweise Duschöle, bekannt, die zur gleichzeitigen Reinigung und Rückfettung der Haut eingesetzt werden können. Auch rückfettende Reinigungszubereitungen auf der Basis von Emulsionen sind dem Fachmanne bekannt. So beschreiben die EP 1166772 bzw. die EP 0870495 beispielsweise Reinigungsemulsionen mit einem hohen Ölgehalt.

Derartige Zubereitungen des Standes der Technik weisen jedoch eine Reihe von Nachteilen auf:
■ Das Schaumverhalten der Zubereitungen lässt zu wünschen übrig. Die Zubereitungen schäumen in der Regel schlecht d.h. die Schaummenge ist zu gering, worunter die subjektiv vom Anwender empfundene und die objektive Reinigungsleistung der Zubereitung leiden.
■ Die Verteilbarkeit der Zubereitung auf der Haut ist unbefriedigend. Die meist zähflüssigen, hydrophoben Zubereitungen lassen sich relativ schwer auf der (mit Wasser benetzten) Haut verteilen.
■ Die auf die Haut aufgetragene Zubereitung läßt sich relativ schwer wieder abspülen. Die Ursache hierfür sind ebenfalls die rheologischen Eigenschaften der Zubereitungen und ihre Polarität.
■ Damit die Reinigungszubereitungen auf Emulsionsbasis stabil sind, müssen den Formulierungen große Mengen an Verdickern, beispielsweise Polyacrylate, zugesetzt werden. Größere Mengen an Polyacrylaten verschlechtern jedoch die rheologischen Eigenschaften der Zubereitungen. Die Viskosität wird erhöht, wodurch sich die Zubereitungen schwerer auf der Haut verteilen und nach der Anwendung wieder abspülen lassen. Auch schäumen die Zubereitungen kaum noch. Die Ursache hierfür ist vermutlich die Tatsache, dass die Mischbarkeit mit Wasser bei zunehmenden Gehalt an Polyacrylaten abnimmt. Bei Polyacrylat-Konzentrationen von unter 0,75 Gewichts-% der Zubereitung hingegen, ließen sich bisher keine stabilen Reinigungsemulsionen formulieren.
■ Die rückfettende Wirkung ist mangelhaft.
■ Die Reinigungszubereitungen entfernen gleichzeitig und relativ unselektiv den (lipophilen) Schmutz auf der Haut ebenso wie die hauteigenen Lipide.

Es war daher die Aufgabe der vorliegenden Erfindung, die Mängel des Standes der Technik zu beseitigen oder zumindest zu lindern und eine Reinigungsemulsion zu entwickeln, deren Schaumqualität und Menge deutlich erhöht, deren Verteilbarkeit, Abspülverhalten und Stabilität deutlich verbessert, deren rückfettende Wirkung verstärkt und deren Selektivität bei der Entfernung lipophiler Bestandteile erhöht ist.

Überraschend gelöst wird die Aufgabe durch eine kosmetische und/oder dermatologische Reinigungsemulsion enthaltend
a) Natriumlaurethsulfat und/oder Natriummyrethsulfat in einer Gesamtkonzentration von 2 bis 17 Gewichts-%,
b) ein oder mehrere Polyacrylate, welche gewählt werden aus der Gruppe, die gebildet wird aus anionischen Homo- und/oder Copolymeren der Acrylsäure und/oder alkylierten Acrylsäurederivaten sowie deren Estern, in einer Gesamtkonzentration von 0,20 bis 0,74 Gewichts-%,
c) eine Ölphase in einer Gesamtkonzentration von 43-51 Gewichts-%, enthaltend
   I) Paraffinöl in einer Konzentration von 25 bis 50 Gewichts-% und
   II) ein oder mehrere Öle mit einer Polarität von 5 bis 50 mN/m in einer Gesamtmenge von 1 bis 25 Gewichts-%,
jeweils bezogen auf das Gesamtgewicht der Emulsion, neben gegebenenfalls weiteren kosmetischen und/oder dermatologischen Wirk-, Hilfs- und Zusatzstoffen, die eine Viskosität von 500 bis 3500 mPa s bei 100 1/s aufweist,
sowie
durch die Verwendung einer Wirkstoffkombination aus
a) Natriumlaurethsulfat und/oder Natriummyrethsulfat in einer Gesamtkonzentration von 2 bis 17 Gewichts-%,
b) ein oder mehreren Polyacrylaten, welche gewählt werden aus der Gruppe, die gebildet wird aus anionischen Homo- und/oder Copolymeren der Acrylsäure und/oder alkylierten Acrylsäurederivaten sowie deren Estern, in einer Gesamtkonzentration von 0,20 bis 0,74 Gewichts-%,
c) einer Ölphase in einer Gesamtkonzentration von 43-51 Gewichts-%, enthaltend
   I) Paraffinöl in einer Konzentration von 25 bis 50 Gewichts-% und
   II) ein oder mehreren Ölen mit einer Polarität von 5 bis 50 mN/m in einer Gesamtmenge von 1 bis 25 Gewichts-%,
jeweils bezogen auf das Gesamtgewicht der Emulsion, neben gegebenenfalls weiteren kosmetischen und/oder dermatologischen Wirk-, Hilfs- und Zusatzstoffen mit einer Viskosität von 500 bis 3500 mPa s bei 100 1/s zur Herstellung stabiler kosmetischer und/oder dermatologischer Reinigungsemulsionen mit stark rückfettender Wirkung auf die Haut.

Aufgrund des hohen Ölgehalts wirken diese Zubereitungen darüber hinaus regenerierend in Bezug auf den allgemeinen Hautzustand, vermindern das Trockenheitsgefühl der Haut und machen die Haut geschmeidig.

Die erfindungsgemäße Viskosität wird dabei wie folgt gemessen:
Die Messung der Viskosität erfolgt bei 25°C in einem DIN Zylinder-System bei einer Scherrate von 100 1/s.

Als Maß für die Polarität der lipophilen Komponenten dient erfindungsgemäß die Grenzflächenspannung einer Lipidkomponente gegenüber Wasser. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m wiedergegeben. Sie hat positives Vorzeichen, wenn sie das Bestreben hat, die Grenzfläche zu verkleinern. Im umgekehrten Falle hat sie negatives Vorzeichen.

Die erfindungsgemäße Polarität wurde mit einem KRÜSS Digital-Tensiometer K 10 T, Ring RI 10 bestimmt. Sie kann auch in der dem Fachmann bekannten Weise Tabellenwerken entnommen und berechnet werden.

Die Schaumeigenschaften der Zubereitung ließen sich dabei wie folgt bestimmen:
Die Schaummessungen wurden mit einer modifizierten Variante des Ross-Miles Testes (DIN 53 902) durchgeführt. Diese Abwandlung des Testes wurde von Dr. F.J. Gohlke (Hoechst AG, Frankfurt) in der Zeitschrift Parfümerie und Kosmetik Nr.3/1964, Seiten 59 - 63 beschrieben. Hierbei wird die Versuchsanordnung leicht modifiziert, wodurch sich im Vergleich zur DIN-Vorschrift etwas niedrigere Schaumhöhen ergeben.

Die Modifizierung sieht folgenden Versuchsaufbau vor:
50 ml der zu testenden Tensidlösung, auf 38° temperiert, werden in einen Standzylinder vorgelegt. Aus einer definierten Höhe wird 200 ml Wasser, ebenfalls auf 38° temperiert, mit einem Zusatz von 300 mg/l Calciumsulfat auf die Tensidlösung fallen gelassen. Die hierdurch erzeugte Schaummenge wird sofort, nach 30 Sekunden und nach 1, 3, 5, 10 bzw. 15 Minuten abgelesen und in ein Diagramm eingetragen. Aus den aufgenommenen Daten wird zusätzlich die Schaumstabilität errechnet und ebenfalls grafisch aufgetragen.

Um die rückfettenden Eigenschaften der Zubereitung zu bestimmen, wurde ein Waschtest mit anschließender Lipidanalytik durchgeführt. Die rückfettenden Eigenschaften können dabei durch die Untersuchung der Lipidrückstände auf der Haut bestimmt werden

Die Stabilitätseigenschaften der Zubereitung ließen sich dabei wie folgt bestimmen:
Als stabil wird eine Formel angesehen, wenn es nach Lagerung bei 40°C über einen Zeitraum von 2 Monaten nicht zur Wasser- und/oder Ölabscheidung kommt.

Erfindungsgemäß bevorzugt ist dabei eine kosmetische und/oder dermatologische Reinigungemulsion, welche die folgenden Konzentrationsbereiche aufweist:
a) Natriumlaurethsulfat und/oder Natriummyrethsulfat in einer Gesamtkonzentration von 4 bis 15 Gewichts-%,
b) ein oder mehrere Polyacrylate, welche gewählt werden aus der Gruppe, die gebildet wird aus an ionischen Homo- und/oder Copolymeren der Acrylsäure und/oder alkylierten Acrylsäurederivaten sowie deren Estern, in einer Gesamtkonzentration von 0,30 bis 0,70 Gewichts-% und besonders bevorzugt von 0,35 bis 0,65 Gewichts-%,
c) Paraffinöl in einer Konzentration von 30 bis 45 Gewichts-% und/oder
d) ein oder mehrere Öle mit einer Polarität von 10 bis 45 mN/m in einer Gesamtmenge von 5 bis 20 Gewichts-%,
jeweils bezogen auf das Gesamtgewicht der Emulsion.

Ebenso ist die Verwendung einer Wirkstoffkombination aus
a) Natriumlaurethsulfat und/oder Natriummyrethsulfat in einer Gesamtkonzentration von 4 bis 15 Gewichts-%,
b) ein oder mehreren Polyacrylaten, welche gewählt werden aus der Gruppe, die gebildet wird aus anionischen Homo- und/oder Copolymeren der Acrylsäure und/oder alkylierten Acrylsäurederivaten sowie deren Estern, in einer von 0,30 bis 0,70 Gewichts-% und besonders bevorzugt von 0,35 bis 0,65 Gewichts-%,
c) Paraffinöl in einer Konzentration von 30 bis 45 Gewichts-% und/oder
d) ein oder mehreren Ölen mit einer einer Polarität von 10 bis 45 mN/m in einer Gesamtmenge von 5 bis 20 Gewichts-%,
jeweils bezogen auf das Gesamtgewicht der Emulsion zur Herstellung stabiler kosmetischer und/oder dermatologischer Reinigungsemulsionen mit stark rückfettender Wirkung auf die Haut, erfindungsgemäß bevorzugt.

Erfindungsgemäß bevorzugt ist ein Gesamtölgehalt von 42-46 Gewichts-% und besonders bevorzugt von 43-45,5 Gewichts-%, jeweils bezogen auf das Gesamtgewicht der Zubereitung.

Ferner ist eine Viskosität der erfindungsgemäßen Zubereitung von 700 bis 3000 mPa s bei 100 1/s erfindungsgemäß bevorzugt.

Erfindungsgemäß vorteilhaft enthalten die erfindungsgemäßen Emulsionen einen Wassergehalt von 35 bis 55 Gewichts-%, wobei ein Wassergehalt von 40 bis 50 Gewichts-% (jeweils bezogen auf das Gesamtgewicht der Zubereitung) erfindungsgemäß bevorzugt ist.

Ein erfindungsgemäß vorteilhaftes Natriumlaurethsulfat ist beispielsweise Genapol LRO Paste von Clariant, Texapon N 70 von Cognis oder Elfan NS 242 Conc. von Akzo Nobel.

Ein erfindungsgemäß vorteilhaftes Natriummyrethsulfat ist beispielsweise Texapon 14S Special von Cognis oder Sulfochem ME-60 von Chemron.

Ferner ist es erfindungsgemäß bevorzugt, als Polyacrylat Carbopol ETD 2020 von Noveon einzusetzen. Dieses weist die folgende Produkt Spezifikationen auf: Viskosität bei einem pH Wert von 5,8-6,3, bei 1% Einsatzkonzentration in Wasser, Brookfield RVT, 20 rpm bei 25°C 47000 - 77000 mPa s.

Vorteilhaft ist es auch, wenn das eingesetzte Polyacrylat bei einer Einsatzkonzentration von 1% und einem pH Wert von 5,2-5,6 in einer 10%igen wässrigen Natrium Laurylethersulfatlösung bei 25°C eine Viskosität von 500 - 1000 mPa s bei einer Scherrate von 100 1/s besitzt.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Erfindungsgemäß besonders bevorzugt sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Carbopol ETD 2020, Carbopol 3128, Pemulen TR1 und Pemulen TR2 bei der NOVEON Inc. erhältlichen.

Als erfindungsgemäß vorteilhaft sind die erfindungsgemäßen bzw. erfindungsgemäß verwendeten Emulsionen anzusehen, die dadurch gekennzeichnet sind, dass ein oder mehrere Öle mit einer Polarität von 5 bis 50 mN/m enthalten.

Erfindungsgemäß vorteilhafte Bestandteile der Ölphase der erfindungsgemäßen Zubereitung werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner können eine oder mehrere Ölkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Son nenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 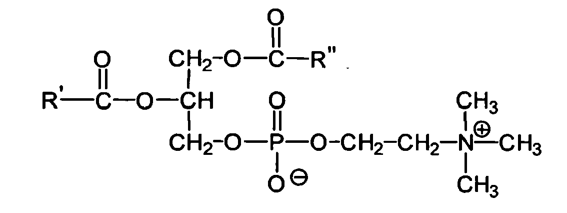 auszeichnen, wobei R' und R" typischerweise unverzweigte aliphatische Reste mit 15 oder 17 Kohlenstoffatomen und bis zu 4 cis-Doppelbindungen darstellen.

Als erfindungsgemäß vorteilhaftes Paraffinöl kann erfindungsgemäß Merkur Weissoel Pharma 40 von Merkur Vaseline, Shell Ondina 917, Shell Ondina 927, Shell Oil 4222, Shell Ondina 933 von Shell & DEA Oil, Pionier 6301 S, Pionier 2071 von Hansen & Rosenthal eingesetzt werden.

Erfindungsgemäß vorteilhaft ist ein Gesamtgehalt an lipophilen Komponenten (Paraffinöl und polare Öle) von 43 bis 50 Gewichts -%, bezogen auf das Gesamtgewicht der Zubereitung.

Erfindungsgemäße Emulsion beziehungsweise Emulsionen, die erfindungsgemäß verwendet werden, sind ferner dadurch gekennzeichnet, dass das Gewichtsverhältnis der Gesamtmengen von Polyacrylaten und Paraffinöl vorteilhaft von 1:125 bis 1:68 und bevorzugt von 1:100 bis 1:57 beträgt.

Die Zusammensetzungen enthalten gemäß der Erfindung außer den vorgenannten Substanzen gegebenenfalls die in der Kosmetik oder Dermatologie üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Darüber hinaus können die erfindungsgemäßen Zubereitungen weitere Tenside enthalten. Es ist erfindungsgemäß von Vorteil, wenn als Tenside anionische, kationische, nichtionische und/oder amphotere Tenside eingesetzt werden.

Unter erfindungsgemäßen Zubereitungen werden in dieser Schrift sowohl Zubereitungen verstanden, die erfindungsgemäß zusammengesetzt sind, als auch Zubereitungen die eine erfindungsgemäß verwendete Wirkstoffkombination enthalten.

Erfindungsgemäß bevorzugt werden ionische Tenside, d.h. anionische, kationische und/oder amphotere Tenside eingesetzt.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.
   Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
   Weitere vorteilhafte kationische Tenside im Sinne der vorliegenden Erfindung sind ferner
■ Alkylamine,
■ Alkylimidazole und
■ ethoxylierte Amine
und insbesondere deren Salze.

Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.
Weitere vorteilhafte nicht-ionische Tenside sind Alkohole und Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß von Vorteil, wenn ein oder mehrere dieser Tenside in einer Konzentration von 1 bis 30 Gewichts-%, bevorzugt in einer Konzentration 5 von 25 bis Gewichts-% und ganz besonders bevorzugt in einer Konzentration von 10 bis 20 Gewichts-%, jeweils bezogen auf das Gesamtgewicht der Zubereitung eingesetzt wird.

Ferner können Polysorbate als waschaktive Agentien erfindungsgemäß vorteilhaft in die Zubereitung eingearbeitet werden.
Im Sinne der Erfindung vorteilhafte Polysorbate sind dabei das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).
   Ganz besonders vorteilhaft sind insbesondere
- Polyoxyethylen(20)sorbitanmonopalmitat (Tween 40, CAS-Nr. 9005-66-7)
- Polyoxyethylen(20)sorbitanmonostearat (Tween 60, CAS-Nr. 9005-67-8).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Formulierung einzeln oder als Mischung mehrer Polysorbate, eingesetzt.

Vorteilhaft kann der Zusatz von Konditionierungsmitteln sein. Dazu zählen polymeren quatären Ammoniumverbindungen, auch können kationische Cellulosederivate und Polysaccharide eingesetzt werden. Weiterhin werden auch Silikonverbindungen zur Konditionierung eingesetzt.

Erfindungsgemäß vorteilhafte Konditionierer können dabei aus den in der Tabelle aufgelisteten Verbindungen gewählt werden.

**Tabelle 1: Erfindungsgemäß vorteilhafte Filmbildner**

| **Bezeichnung nach INCI** | **CAS-Nummer** | **Polymertyp** | **Beispiel (Handelsname)** |
|---|---|---|---|
| Polyquaternium-2 | CAS 63451-27-4 | Urea, N, N'- bis[3-(dimethylamino)propyl]- , polymer mit 1, 1'-oxybis(2- chloroethan) | Mirapol® A-15 |
| Polyquaternium-5 | CAS 26006-22-4 | Acrylamid, β-Methacryloxyethyltriethyl ammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat® S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SC-230M, Polymer JR 400 |
| Polyquaternium-11 | CAS 53633-54-8 | Vinylpyrrolidon/dimethyla minoethyl-Methacrylat-Copolymer/Diethylsulfat-Reaktionsprodukt | Gafquat®755N |
| Polyquaternium-16 | CAS 29297-55-0 | Vinylpyrrolidon/vinylimid azolinummethochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxanpolydimethyldimethylammoniumaceta t-Copolymer | Abil® B 9905 |
| Polyquaternium-22 | CAS 53694-17-0 | Dimethyldiallylammoniu mchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacryl amidopropyltrimethylam moniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/Acryl onitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |

Weitere erfindungsgemäß vorteilhafte Konditionierer stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch nichtionische Poly-N-vinylpyrrolidon/Polyvinylacetat-Copolymere (z.B. Luviskol VA 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Konditionierer eingesetzt werden.

Ein Zusatz von Puderrohstoffen kann allgemein vorteilhaft sein. Besonders bevorzugt wird der Einsatz von Talkum.

Ethoxylierte Glycerin-Fettsäureester werden in wässrigen Reinigungsrezepturen zu verschiedenen Zwecken eingesetzt. Niedrig ethoxylierte Glycerin-Fettsäureester (EO 3-12) dienen üblicherweise als Rückfetter zur Verbesserung des Hautgefühls nach dem Abtrocknen, Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt. Allen diesen Substanzen ist gemeinsam, dass sie auf der Haut bei der Anwendung i.e. bei der Verdünnung mit Wasser ein besonderes Hautgefühl erzeugen. Neuartig ist, dass diese Substanzen sowohl die inakzeptable Sensorik beim Waschen (verursacht durch den Gelbildner) neutralisieren, als auch nach dem Abtrocknen einen für den Verbraucher angenehmes Hautgefühl verursachen. Sie wirken nicht nur der Austrocknung durch die hohen Tensidmengen entgegen, sondern verbessern die Sensorik noch merklich.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Überraschend wurde gefunden, dass sich in die erfindungsgemäßen Reinigungsemulsionen verschiedenste Wirkstoffe mit unterschiedlicher Löslichkeit homogen einarbeiten lassen. Die Substantivität der Wirkstoffe auf Haut und Haar ist aus der beschriebenen Reinigungsemulsion wesentlich höher als aus herkömmlichen tensidhaltigen Reinigungsformulierungen. Es ist zu vermuten, dass die Auswaschung der Wirkstoffe von der Haut durch die in der Formel enthaltenen Tenside durch die Bildung eines Ölfilms auf der Haut vermindert oder zumindest verringert wird, so dass eine größere Menge der im Produkt enthaltenen Wirkstoffe auf der Haut verbleibt.

Erfindungsgemäß können die Wirkstoffe (eine oder mehrere Verbindungen) sehr vorteilhaft gewählt werden aus der Gruppe der Wirkstoffe, insbesondere aus folgender Gruppe:

Acetylsalicylsäure, Atropin, Azulen, Hydrocortison und dessen Derivaten, z. B. Hydrocortison-17-valerat, Vitamine der B- und D-Reihe, sehr günstig das Vitamin B₁, das Vitamin B ₁₂ das Vitamin D₁, Vitamin A bzw. dessen Derivate wie Retinylpalmitat, Vitamin E oder dessen Derivate wie z.B. Tocopheryl Acetat, Vitamin C und dessen Derivate wie z.B. Ascorbylglucusid aber auch Niacinamid, Panthenol, Bisabolol, Polydocanol, ungesättigte Fettsäuren, namentlich die essentiellen Fettsäuren (oft auch Vitamin F genannt), insbesondere die γ-Linolensäure, Ölsäure, Eicosapentaensäure, Docosahexaensäure und deren Derivate, Chloramphenicol, Coffein, Prostaglandine, Thymol, Campher, Squalen, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen, Weihrauchextrakt, Grünteeextrakt, Wasserlilienextrakt, Süßholzextrakt, Hamamelis, Antischuppenwirkstoffe (z.B. Selendisulfid, Zinkpyrithion, Pirocton, Olamin, Climbazol, Octopirox, Polydocanol und deren Kombinatinen), Komplexwirkstoffen wie z.B. jenen aus γ-Oryzanol und Calciumsalzen wie Calciumpanthotenat, Calciumchlorid, Calciumacetat.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wässrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydrooyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben): 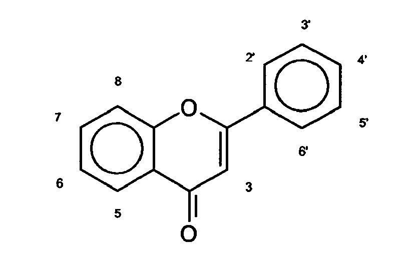 Einige der wichtigeren Flavone, welche auch bevorzugt in erfindungsgemäßen Zubereitungen eingesetzt werden können, sind in der nachstehenden Tabelle 2 aufgeführt:

| **Tabelle 2** | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 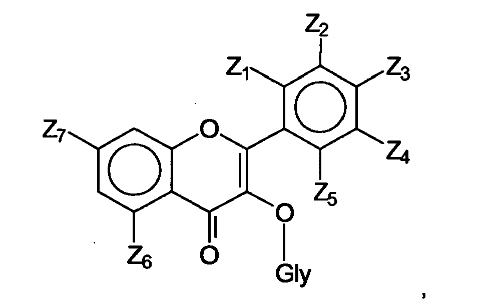 wobei Z₁ bis Z₇ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 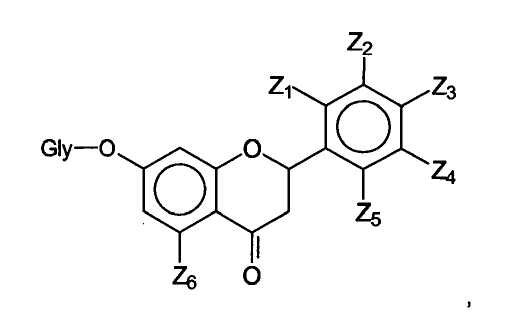 wobei Z₁ bis Z₆ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 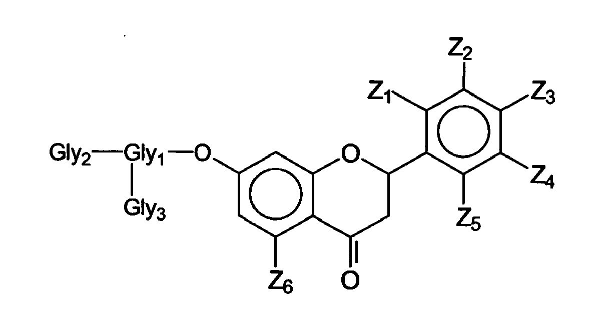 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur 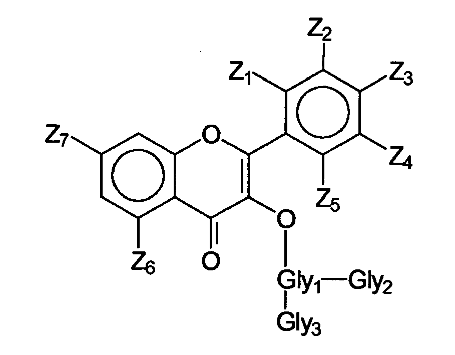 Besonders vorteilhaft werden die erfindungsgemäßen Flavonglycoside aus der Gruppe, welche durch die folgende Struktur wiedergegeben werden: 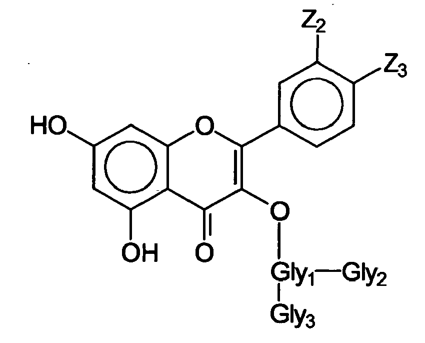 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ un abhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 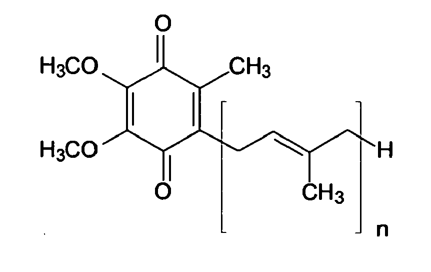 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

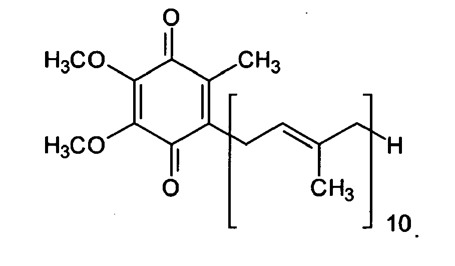

Plastochinone weisen die allgemeine Strukturformel 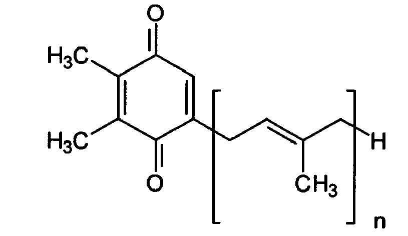 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus: 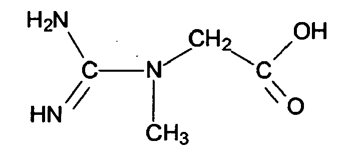 Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 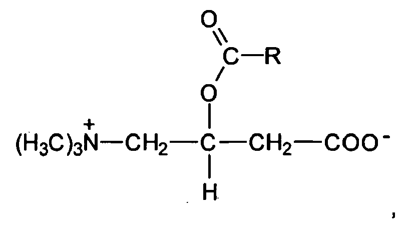 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Außerdem können die erfindungsgemäßen Wirkstoffe (eine oder mehrere Verbindungen) auch sehr vorteilhaft gewählt werden aus der Gruppe der hydrophilen Wirkstoffe, insbesondere aus folgender Gruppe:
Alpha Hydroxy Säuren wie Milchsäure oder Salicylsäure bzw. deren Salze wie z.B. Na-Lactat, Ca-Lactat, TEA-Lactat, Harnstoff, Allantoin, Serin, Sorbitol, Glycerin, Milchproteine, Panthenol, Chitosan.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2 -Chloracetamid, Benzalkoniumchlorid, Benzylalkohol, Salicylsäure und Salicylate.

Es ist dabei erfindungsgemäß besonders bevorzugt, wenn als Konservierungsstoffe lodopropylbutylcarbamate, Parabene (Methyl-, Ethyl-, Propyl- und/oder Butylparaben) und/oder Phenoxyethanol eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung enthalten.

Die erfindungsgemäße Zubereitung enthält vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Alle in dieser Schrift erwähnten Konzentrationsangaben beziehen sich auf das Gesamtgewicht der Reinigungsemulsion, sofern keine abweichenden Bezugsgrößen explizit erwähnt sind.

Erfindungsgemäß vorteilhaft kann die erfindungsgemäße Zubereitung Glittersoffe und/oder andere Effektstoffe (z.B. Farbschlieren) enthalten.

Es ist erfindungsgemäß vorteilhaft, wenn die erfindungsgemäße kosmetische Zubereitung in einer Flasche oder Quetschflasche aufbewahrt und aus dieser heraus angewendet werden. Entsprechend sind auch Flaschen oder Quetschflaschen, welche eine erfindungsgemäße Zubereitung enthalten, erfindungsgemäß.

Neben Quetschflaschen kann die erfindungsgemäße Emulsion auch vorteilhaft in Tuben aufbewahrt und aus diesen heraus angewendet werden. Auch die Aufbewahrung in einem 2-Kammer-Packmittel ist erfindungsgemäß vorteilhaft.

Erfindungsgemäß vorteilhaft kann die erfindungsgemäße Zubereitung als Tränkung auf ein Substrat aufgetragen sein. Die erfindungsgemäßen Substrate können glatt oder auch oberflächenstrukturiert sein. Erfindungsgemäß bevorzugt sind oberflächenstrukturierte Substrate.

Erfindungsgemäß ist auch die Kombination aus der erfindungsgemäßen kosmetischen und/oder dermatologischen Zubereitung und einem unlöslichen Substrat.

Erfindungsgemäß bevorzugt werden in einem solchen Falle Substrate in Form von Tüchern eingesetzt, welche aus Vlies bestehen, insbesondere aus wasserstrahlverfestigten und/oder wasserstrahlgeprägten Vlies. Die Substrate können vorteilhaft auch als Bausch, gelochtes Vlies oder Netz ausgeführt sein.

Erfindungsgemäß vorteilhaft kann die erfindungsgemäße Emulsion in Form einer mit einem Gas aufgeschäumten Creme (auch Mousse genannt) beispielsweise in einem Tiegel vorliegen.

Erfindungsgemäß vorteilhaft kann die erfindungsgemäße Zubereitung in einer Druckgasflasche oder Aerosoldose vorliegen. Erfindungsgemäß bevorzugt sind dabei Druckgasflaschen, welche die erfindungsgemäße Zubereitung im Inneren eines flexiblen Beutels enthalten, dessen Inhalt durch den Überdruck, welcher in der Druckgasflasche auf den Beutel wirkt, nach außen gedrückt wird. Besonders bevorzugt enthält die Zubereitung in einem solchen Falle eine weitere leichtflüchtige Komponente wie Pentan, wodurch sich so genannte "nachschäumende" Zubereitungen herstellen lassen.

Erfindungsgemäß vorteilhaft ist auch die Aufbewahrung und Anwendung der erfindungsgemäßen Zubereitung mit einer Pumpspray-Apparatur oder mit einem Pumpspender ("Pumpfoamer").

Erfindungsgemäß ist die Verwendung der kosmetischen oder dermatologischen Reinigungsemulsionen als Schaum-, Dusch- oder Wannenbad, Gesichtsreiniger sowie als Haarshampoo.

Erfindungsgemäß ist ferner die Verwendung zur Reinigung der Haut und ihrer Anhangsgebilde (z.B. Haare, Nägel).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispielrezepturen**

| | **1** | **2** | **3** | **4** | **5** |
|---|---|---|---|---|---|
| Natriumlaurethsulfat | 7,00 | 10,00 | - | 9 | - |
| Cocamidopropylbetain | 3,00 | - | - | 2 | 3 |
| Natrium Myristylethersulfat | - | - | 5 | - | 6 |
| Alkylpolyglucoside | 2,00 | - | - | - | 2,5 |
| Polyacrylat | 0,50 | 0,60 | 0,7 | 0,35 | 0,45 |
| PEG-7 Glyceryl Cocoate | 1,00 | - | 1,00 | - | - |
| Quaternäres Ammoniumsalz der Hydroxyethylcellulose | - | - | - | 0,10 | - |
| Talkum | 0,50 | - | - | - | - |
| Paraffinöl | 25 | 35 | 40 | 30 | 45 |
| Sojaöl | 18 | 6 | 5 | 13 | - |
| Mandelöl | - | 1 | - | - | - |
| Jojobaöl | - | 1 | - | - | 0,5 |
| Unispheres (Lactose + Cellulose + Hydroxypropyl Methylcellulose + Cl 77007) | - | 0,2 | - | - | - |
| Phenoxethanol + Methylparaben + Butylparaben + Ethylparaben + Isobutylparaben + Propylparaben | 1,00 | 0,8 | 1,00 | 1,00 | 1,00 |
| Butylhydroxytoluol | 0,05 | 0,05 | 0,05 | 0,05 | - |
| NaOH | q.s. | q.s. | q.s. | q.s. | q.s. |
| Parfum | 1,5 | 1,2 | 1 | 1 | 1,2 |
| Wasser | ad 100 | ad 100 | ad 100 | ad 100 | ad 100 |

## Patentansprüche

1. Kosmetische und/oder dermatologische Reinigungemulsion enthaltend
a) Natriumlaurethsulfat und/oder Natriummyrethsulfat in einer Gesamtkonzentration von 2 bis 17 Gewichts-%,
b) ein oder mehrere Polyacrylate, welche gewählt werden aus der Gruppe, die gebildet wird aus anionischen Homo- und/oder Copolymeren der Acrylsäure und/oder alkylierten Acrylsäurederivaten sowie deren Estern, in einer Gesamtkonzentration von 0,20 bis 0,74 Gewichts-%,
c) eine Ölphase in einer Gesamtkonzentration von 43-51 Gewichts-%, enthaltend
I) Paraffinöl in einer Konzentration von 25 bis 50 Gewichts-% und
II) ein oder mehrere Öle mit einer Polarität von 5 bis 50 mN/m in einer Gesamtmenge von 1 bis 25 Gewichts-%,
jeweils bezogen auf das Gesamtgewicht der Emulsion, neben gegebenenfalls weiteren kosmetischen und/oder dermatologischen Wirk-, Hilfs- und Zusatzstoffen, die eine Viskosität von 500 bis 3500 mPa s bei 100 1/s aufweist.

2. Verwendung einer Wirkstoffkombination aus
a) Natriumlaurethsulfat und/oder Natriummyrethsulfat in einer Gesamtkonzentration von 2 bis 17 Gewichts-%,
b) ein oder mehreren Polyacrylaten, welche gewählt werden aus der Gruppe, die gebildet wird aus anionischen Homo- und/oder Copolymeren der Acrylsäure und/oder alkylierten Acrylsäurederivaten sowie deren Estern, in einer Gesamtkonzentration von 0,20 bis 0,74 Gewichts-%,
c) einer Ölphase in einer Gesamtkonzentration von 43-51 Gewichts-%, enthaltend
I) Paraffinöl in einer Konzentration von 25 bis 50 Gewichts-% und
II) ein oder mehreren Ölen mit einer Polarität von 5 bis 50 mN/m in einer - Gesamtmenge von 1 bis 25 Gewichts-%,
jeweils bezogen auf das Gesamtgewicht der Emulsion, neben gegebenenfalls weiteren kosmetischen und/oder dermatologischen Wirk-, Hilfs- und Zusatzstoffen mit einer Viskosität von 500 bis 3500 mPa s bei einer Scherrate von 100 1/s bei 25°C, zur Herstellung stabiler kosmetischer und/oder dermatologischer Reinigungsemulsionen mit stark rückfettender Wirkung auf die Haut.

3. Emulsion beziehungsweise Verwendung nach einem der vorhergehenden Ansprüche, **dadurch gekennzeichnet, dass** ein oder mehrere Öle mit einer Polarität von 5 bis 50 mN/m gewählt werden aus der Gruppe der Kohlenwasserstoffe, der Fettsäuretriglyceride, der Silikonöle und der Carbonsäureester.

4. Emulsion beziehungsweise Verwendung nach einem der vorhergehenden Ansprüche, **dadurch gekennzeichnet, dass** das Gewichtsverhältnis der Gesamtmengen von Polyacrylaten und Paraffinöl von 1:125 bis 1:68 beträgt.

## Claims

1. A cosmetic and/or dermatological cleansing emulsion comprising
a) sodium laureth sulfate and/or sodium myreth sulfate in a total concentration of 2 to 17% by weight,
b) one or more polyacrylates, which are chosen from the group which is formed from anionic homo- and/or copolymers of acrylic acid and/or alkylated acrylic acid derivatives and their esters, in a total concentration of 0.20 to 0.74% by weight,
c) an oil phase in a total concentration of 43-51 % by weight, comprising
I) paraffin oil in a concentration of 25 to 50% by weight and
II) one or more oils having a polarity of 5 to 50 mN/m in a total amount of 1 to 25% by weight,
in each case based on the total weight of the emulsion, in addition optionally further cosmetic and/or dermatological active ingredients, excipients and additives, which has a viscosity of 500 to 3500 mPa s at 100 1/s.

2. The use of an active ingredient combination consisting of
a) sodium laureth sulfate and/or sodium myreth sulfate in a total concentration of 2 to 17% by weight,
b) one or more polyacrylates, which are chosen from the group which is formed from anionic homo- and/or copolymers of acrylic acid and/or alkylated acrylic acid derivatives and their esters, in a total concentration of 0.20 to 0.74% by weight,
c) an oil phase in a total concentration of 43-51 % by weight, comprising
I) paraffin oil in a concentration of 25 to 50% by weight and
II) one or more oils having a polarity of 5 to 50 mN/m in a total amount of 1 to 25% by weight,
in each case based on the total weight of the emulsion, in addition optionally further cosmetic and/or dermatological active ingredients, excipients and additives, having a viscosity of 500 to 3500 mPa s at a shear rate of 100 1/s at 25°C, for the production of stable cosmetic and/or dermatological cleansing emulsions having strongly refatting action on the skin.

3. An emulsion or use as claimed in one of the preceding claims, **characterized in that** one or more oils having a polarity of 5 to 50 mN/m is/are selected from the group consisting of the hydrocarbons, the fatty acid triglycerides, the silicone oils and the carboxylic acid esters.

4. An emulsion or use as claimed in one of the preceding claims, **characterized in that** the weight ratio of the total amounts of polyacrylates and paraffin oil is from 1:125 to 1:68.

## Revendications

1. Emulsion nettoyante cosmétique et/ou dermatologique, contenant
a) du laurethsulfate de sodium et/ou du myrethsulfate de sodium en une concentration totale de 2 à 17% en poids,
b) un ou plusieurs polyacrylates, qui sont choisis dans le groupe formé par les homopolymères et/ou les copolymères anioniques de l'acide acrylique et/ou des dérivés alkylés de l'acide acrylique ainsi que de leurs esters, en une concentration totale de 0,20 à 0,74% en poids
c) une phase huileuse en une concentration totale de 43-51% en poids contenant
I) de l'huile de paraffine en une concentration de 25 à 50% en poids et
II) une ou plusieurs huiles présentant une polarité de 5 à 50 mN/m en une quantité totale de 1 à 25% en poids,
à chaque fois par rapport au poids total de l'émulsion, en plus, le cas échéant, d'autres substances actives, adjuvants et/ou additifs cosmétiques et/ou dermatologiques,
qui présente une viscosité de 500 à 3 500 mPa.s à 100 1/s.

2. Utilisation d'une combinaison de substances actives constituée par
a) du laurethsulfate de sodium et/ou du myrethsulfate de sodium en une concentration totale de 2 à 17% en poids,
b) un ou plusieurs polyacrylates, qui sont choisis dans le groupe formé par les homopolymères et/ou les copolymères anioniques de l'acide acrylique et/ou des dérivés alkylés de l'acide acrylique ainsi que de leurs esters, en une concentration totale de 0,20 à 0,74% en poids
c) une phase huileuse en une concentration totale de 43-51% en poids contenant
I) de l'huile de paraffine en une concentration de 25 à 50% en poids et
II) une ou plusieurs huiles présentant une polarité de 5 à 50 mN/m en une quantité totale de 1 à 25% en poids,
à chaque fois par rapport au poids total de l'émulsion, en plus, le cas échéant, d'autres substances actives, adjuvants et/ou additifs cosmétiques et/ou dermatologiques,
présentant une viscosité de 500 à 3 500 mPa.s à une vitesse de cisaillement 100 1/s à 25°C
pour la préparation d'émulsions de nettoyage cosmétiques et/ou dermatologiques stables avec un effet renourrissant important sur la peau.

3. Emulsion ou utilisation selon l'une quelconque des revendications précédentes, **caractérisée en ce qu'**une ou plusieurs huiles présentant une polarité de 5 à 50 mN/m sont choisies dans le groupe des hydrocarbures, des triglycérides d'acide gras, des huiles de silicone et des esters d'acide carboxylique.

4. Emulsion ou utilisation selon l'une quelconque des revendications précédentes, **caractérisée en ce que** le rapport pondéral des quantités totales de polyacrylates et d'huile de paraffine est de 1:125 à 1:68.
